# EUROPEAN PATENT APPLICATION

(11) **EP 4 265 721 A1**
(43) Date of publication of application: **25.10.2023**
(21) Application number: 21906647.9
(22) Date of filing: 15.12.2021
(51) Int. Cl.: C12N 15/09, C12Q 1/6844, C12Q 1/6869

(54) **METHOD FOR EVALUATING ADAPTER LIGATION EFFICIENCY IN SEQUENCE OF DNA SAMPLE**

(30) Priority: 15.12.2020 JP 2020207319
(71) Applicant: Genodive Pharma Inc., Atsugi-shi, Kanagawa, 243-0018 (JP)
(72) Inventor: TANAKA, Masafumi, Isehara-shi, Kanagawa 259-1142 (JP); INOKO, Hidetoshi, Yokohama-shi, Kanagawa 245-0002 (JP)
(74) Representative: Symbiosis IP Limited
(86) International application number: PCT/JP2021/046211
(87) International publication number: WO 2022/131285

(57) **Abstract**

The purpose of the present invention is to provide a method for conveniently and accurately evaluating the ligation efficiency in the DNA sequencing process in order to optimize the condition of ligating Y-type adapters to both ends of a double-stranded DNA fragment. The present invention relates to a method for evaluating the efficiency of ligation reaction through which Y-type adapters are ligated to both ends of DNA to be analyzed, in the sequencing process of DNA to be analyzed using the Y-type adapter, wherein the efficiency of reaction is evaluated by electrophoresing a reaction mixture containing ligation molecules, between the DNA and the Y-type adapters, produced by the ligation reaction under a specified condition, and analyzing a band separated on the basis of the number of adapters ligated to the DNA.

## Description

### Technical Field

The present invention relates to a method for evaluating efficiency of an adapter ligation in order to optimize the condition for the adapter ligation in DNA sequencing using double-stranded barcode adapters. The method is especially useful for DNA sequencing of specimen that contains a scarce amount of DNA sample, such as the specimen used in a liquid biopsy.

### Background Art

Conventionally, a cancer diagnosis has been made by surgically collecting tissue cells (specimen) from a patient with suspected cancer and examining the specimen (biopsy). However, since this examination method is invasive, there have been problems including that a timely follow-up is not possible considering the burden on the patient.

On the other hand, free DNA (cell-free DNA (cfDNA)) released from tissue cells by processes such as apoptosis is present in the blood. In the case of a cancer patient, cfDNA derived from cancer cells is also contained in the blood. In recent years, researches have made progress on using the cfDNA derived from cancer cells for cancer diagnosis, attracting attention as a minimally invasive examination method known as "a liquid biopsy".

In the examination method using a liquid biopsy, cfDNA derived from cancer cells contained in the peripheral blood of a cancer patient is sequenced using a so-called next-generation sequencer and presence or absence of mutations characteristic of cancer cells is detected, thereby enabling cancer diagnosis in a convenient and minimally invasive manner. However, it is said that 1 mL of blood, either from a healthy person or a cancer patient, contains cfDNA corresponding to only about 1000 molecules of human genome. In the case of a cancer patient, only a part of the cfDNA is derived from cancer cells. Sequencing by the use of such a scarce amount of DNA sample requires improvement in precision of the sequencing as much as possible.

As a device to be used for sequencing a DNA sample, a next-generation sequencer (NGS) is generally used. However, the error rate of the next generation sequencer currently used is said to be about 0.1%. A method for improving the read precision in the next generation sequencer such as a massively parallel sequencer includes molecular barcode technology. In the molecular barcode technology, PCR amplification is performed on the target DNA to which a molecular barcode generating sequence was added in prior. Since nucleotide sequences having the same molecular barcode are derived from the same molecule, the read error can be eliminated by generating a consensus sequence thereof.

For adding the barcode sequence to cfDNA, there is a method of using a primer and another method of attaching an adapter with a barcode generating sequence embedded therein by ligation.

In Patent Document 1, it is described that a Y-shaped adapter containing a unique molecular index (UMI), i.e., a barcode generating sequence, was added to both ends of a double-stranded DNA fragment by ligation, and PCR amplification was performed, enabling sequencing with excellent accuracy and sensitivity, unaffected by errors and noises. In addition, in Patent Document 2, a new Y-type (fork-type) adapter with reduced error rate is proposed.

However, according to the sequencing method as described in Patent Document 1, accurate sequence information cannot be obtained unless two Y-type adapters are completely bound (ligated) to both ends of a double-stranded DNA fragment (sample) via phosphodiester bonds at four sites. In particular, in order to apply the "double-stranded nucleotide sequencing" (Duplex Sequencing), which is a UMI-based sequence reconstruction of each starting double-stranded source molecule, it is prerequisite to obtain sequence information for both strands, and it is essential that the adapter molecule ligation is complete. In general, the ligation of DNA fragments to adapters is performed using an enzyme called DNA ligase, but in most cases reaction conditions recommended by raw material (enzyme) suppliers are applied, and there is no method for conveniently measuring the reaction efficiency.

In particular, when sequencing based on a scarce amount of DNA sample such as cfDNA, it is necessary to minimize waste of the DNA sample as much as possible. Therefore, a method for evaluating efficiency of the adapter ligation conveniently and accurately is required to optimize the condition for the ligation reaction.

### Citation List

### Patent Document

Patent Document 1: JP-B 6685324
Patent Document 2: JP-A 2019-504624

### Summary of Invention

### Technical Problem

In order to optimize the condition for binding (ligating) Y-type (Y-shaped) adapters to both ends of a double-stranded DNA fragment in sequencing a DNA sample, the present invention is intended to provide a method for evaluating efficiency of the ligation conveniently and accurately.

### Solution to Problem

The present inventors created a model DNA fragment (double-stranded) and model Y-type adapters, and purposefully prepared a model ligation molecule with adapters ligated to one to four sites at the four ends of the model double-stranded DNA fragment. Furthermore, they verified for the first time that the prepared model ligation molecule can be classified (identified) according to the number of adapter ligations by electrophoresis. Based on these findings, the present inventors established a method for evaluating efficiency of binding (ligation) reaction between a DNA fragment to be analyzed in DNA sequencing and Y-type adapters by mobility of the produced ligation molecule in electrophoresis. The evaluation method can be used to optimize the condition for the ligation reaction using a DNA ligase.

That is, the present invention provides a method for evaluating efficiency of a ligation reaction that ligates Y-type adapters to both ends of DNA to be analyzed, in sequencing the DNA to be analyzed using the Y-type adapters, wherein the efficiency of the reaction is evaluated by electrophoresing a reaction mixture containing ligation molecules comprising the DNA and the Y-type adapters produced by the ligation reaction under a specified condition, and analyzing a band separated based on the number of adapters ligated to the DNA.

In addition, the present invention provides a method for optimizing a condition for a ligation reaction, comprising the steps of: (1) performing the ligation reaction under a first specified condition and performing the evaluation method on the reaction mixture to evaluate a first reaction efficiency; then (2) performing the ligation reaction under a second specified condition which is at least partially modified from the first specified condition, and performing the method of evaluation on the reaction mixture to evaluate a second reaction efficiency; and (3) comparing the first reaction efficiency with the second reaction efficiency. In this method for optimization, the steps (2) and (3) repeatedly multiple times can be performed to find the optimal reaction condition.

### Advantageous Effects of Invention

According to the method of the present invention, the reaction efficiency in the reaction that binds (ligates) the Y-type adapters to the DNA molecule to be analyzed can be evaluated by a simple operation. Therefore, by performing the evaluation method of the present invention while modifying the reaction condition, the condition for a highly efficient and complete ligation of the Y-type adapters to the DNA molecule can be found conveniently and accurately.

The evaluation method of the present invention is effective in sequencing using a liquid biopsy. Also, in sequencing of genome and DNA in general, it is essential to create a library before performing the sequencing. Therefore, the technique for creating a library using the evaluation method of the present invention can be applied not only to a liquid biopsy but also to sequencing of genome and DNA in general, thus contributing to the improvement of efficiency, accuracy and precision of sequencing.

### Brief description of Drawings

[FIG. 1] FIG. 1 is a schematic diagram illustrating the step of sequencing DNA to be analyzed (double strands) using Y-type adapters.
[FIG. 2] FIG. 2 is a schematic diagram showing structures of molecules that can be produced in the ligation reaction between DNA molecules and Y-type adapters.
[FIG. 3] FIG. 3 is a schematic diagram showing model molecules used to measure efficiency of the ligation reaction between DNA molecules and Y-type adapters.
[FIG. 4] FIG. 4 is a schematic diagram showing combinations of DNA molecules and Y-type adapters, and structures of ligation molecules produced by the ligation reaction in these combinations.
[FIG. 5] FIG. 5 is a diagram showing the results of electrophoresis for separation based on the structures of ligation molecules (number of adapters ligated).
[FIG. 6] FIG. 6 is a diagram showing the electrophoretic distribution of ligation molecules obtained from ligation reactions by changing the reaction conditions of the ligation reactions using commercially available kits.
[FIG. 7] FIG. 7 is a diagram showing the distribution of ligation molecules obtained from ligation reactions using cfDNA samples.

### Description of Embodiments

The present invention is described in detail below.

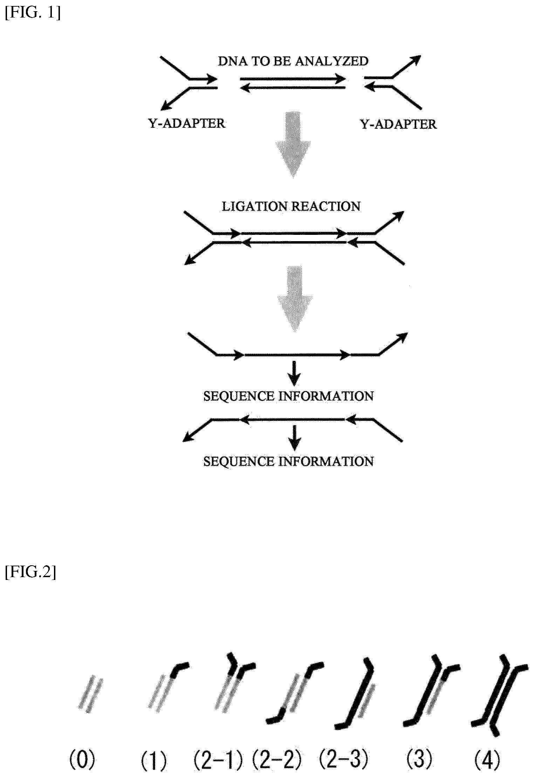

In large-scale sequencing using a next-generation sequencer (NGS), when sequencing DNA to be analyzed using Y-type adapters, the Y-type adapters are bound (ligated) to both ends of the DNA to be analyzed (double strands), as shown in FIG. 1.

If the adapter molecules corresponding to both ends of each DNA strand are properly bound (ligated) through phosphodiester bonds for the upper DNA strand and the lower DNA strand of the DNA to be analyzed, both strands will be amplified by PCR and sequence information of the relevant DNA strand can be obtained. However, a DNA strand to which the adapter molecules were not ligated properly will not allow the PCR reaction to proceed, making proper sequence information unavailable.

However, there is no guarantee that the adapter ligation reaction is complete, and in principle, incomplete adapter ligation molecules may be produced in which (an) adapter molecule(s) is/are not ligated to one or both end(s) of each DNA strand. That is, it is expected that a sample after the binding (ligation) reaction contains both of incomplete and complete adapter ligation molecules as shown in FIG. 2. The numbers shown in parentheses in FIG. 2 (the first number in parentheses) are the number of adapter molecules ligated to the DNA molecule. That is, the resultant molecules include: a DNA molecule to which no adapter is ligated at all (0), a DNA molecule to which one adapter molecule is ligated to only one end of one of DNA strands (1), DNA molecules to which adapter molecules are ligated to only one end of both of DNA strands (2-1 and 2-2), a DNA molecule to which adapter molecules are ligated to both ends of only one of DNA strands (2-3), a DNA molecule to which an adapter is not ligated to only one end of one of DNA strands (3) (all of these are also referred to as "incomplete adapter ligation molecules"), and a DNA molecule to which adapter molecules are ligated to both ends of both of DNA strands (4: also referred to as "complete adapter ligation molecule"). Also, sequence information can be obtained on only the DNA strands shown in dark color in FIG. 2. Therefore, the production of incomplete adapter ligation molecules in the binding (ligation) reaction contributes to a decrease in sequencing efficiency.

Therefore, it is extremely important to allow to efficiently produce "complete adapter ligation molecules" in the ligation reaction between DNA and adapters. However, at present, there is no convenient method to measure the efficiency of the adapter ligation with high precision, and in most cases, commercially available kits are used under the conditions recommended by the manufacturers of the kits. This invention found for the first time that the electrophoresis can be used for separation based on the number of adapters ligated to DNA, and that the efficiency of production of the "complete adapter ligation molecule" can be evaluated from the results of the electrophoresis.

In order to demonstrate the effectiveness of the evaluation method of the present invention, the present inventors have produced model molecules corresponding to DNA molecules with complete ligation of the four adapters ((4) in FIG. 2; "complete adapter ligation molecule") and DNA molecules with incomplete adapter ligation ((0), (1), (2-1), (2-2), (2-3) and (3) in FIG. 2; "incomplete adapter ligation molecules"). Then, they have verified that separation and identification can be made based on the number of ligated adapters by using these model molecule groups to perform the method of the present invention.

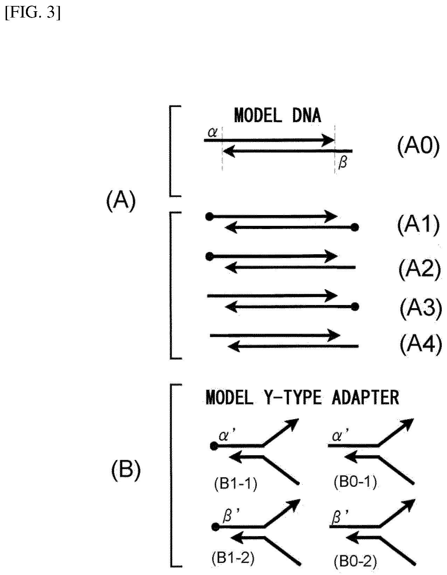

First, model DNA double strands and model Y-type adapters were created as shown in FIG. 3.

For each model molecule preparation, created were model DNA double strands having a different 5' overhang sequence at each end (α and β in FIG. 3(A)) and two types of Y-type adapters each having a 5' overhang sequence (α' and β' in FIG. 3(B)) compatible with each overhang sequence in the model DNA double strands. In addition, by making each overhang sequence an asymmetric sequence, an intermolecular ligation was limited to only predefined ligations. Thus, the ligation product that occurs in a solution containing model DNA double strands and 2 types of model Y-type adapters is limited to a ligation molecule in which either of the model Y-type adapters is ligated to each end of the model DNA double strands, and no ligation occurs between model DNA double strands nor between model Y-type adapters. Furthermore, by designating a specific 5' overhang end as P or OH, it was made possible to prespecify presence or absence of the ligation at the 5' overhang end.

### <Creation of model DNA double strands>

Model DNA double strands were created by PCR. Any PCR strand length can be set, but it was set to 170 bp, the average strand length of cfDNA. A recognition sequence for Type IIS restriction enzymes such as BsaI and BbsI was added to a primer used in PCR. Therefore, it is possible to produce a predefined 5' overhang end by cleaving the PCR product with these enzymes. Specifically, a PCR product with a BsaI site at one end and a BbsI site at the other end was created, and as appropriate, either or both of the 5' overhang end generated by BsaI and the 5' overhang end generated by BbsI were converted to an OH end by post-cleavage treatment with dephosphorylation enzyme.

The model DNA double strands created are a molecule (A1) having phosphate groups at the 5' ends of both the upper and lower strands of the model DNA molecule (A0), a molecule (A2) having a phosphate group only at the 5' end of the upper strand (α portion), a molecule (A3) having a phosphate group only at the 5' end of the lower strand (β portion), and a molecule (A4) having no phosphate group at the 5' end of the upper strand or lower strand.

### <Creation of model Y-type adapters>

Model Y-type adapters with an adapter sequence for a sequencer by Illumina Inc. and the 5' overhang end phosphorylated were synthesized.

As outlined in FIG. 3(B), the model Y-type adapter is a Y-type adapter comprising 2 DNA strands that are partially hybridizable to each other, with an overhang at the 5' end of a hybridizable portion. The overhang portion has a nucleotide sequence (α') complementary to the overhang portion (α) of the model DNA molecule or a nucleotide sequence (β') complementary to the overhang portion (β) of the model DNA molecule. Model Y-type adapters (B1-1 and B1-2) each having a phosphate group at the 5' end of the overhang portions (α' and β', respectively) were also synthesized. These model Y-type adapters can be created by synthesizing and annealing a single-stranded DNA. The presence or absence of a phosphodiester bond defined by the presence or absence of phosphate groups can also be defined by creating an adapter with a hydroxyl group at the 5' end, in addition to the dephosphorylation method after enzymatic cleavage described above.

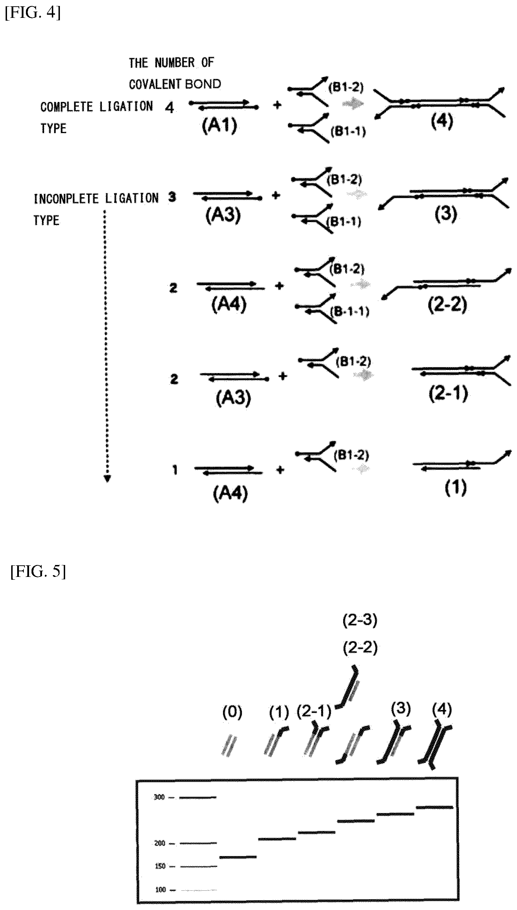

The model DNA double strands and the model Y-type adapters were combined, for example, as described in FIG. 4, and by performing ligation reactions, ligation molecules with structures shown on the right side of FIG. 4 were obtained. These ligation molecules correspond to (4), (3), (2-2), (2-1), and (1), respectively, whose schematic structures are shown in FIG. 2.

Next, a sample containing various ligation molecules described above were electrophoresed using an electrophoresis device, and the mobility (ease of movement) of each ligation molecule was compared. The mobility of these molecules in electrophoresis is affected by complex factors such as the molecular weight of each molecule, shapes of molecules, and electrophoresis conditions including properties of carriers for electrophoresis, and in general, predicting mobility of each molecule is thought to be difficult.

However, when an electrophoresis was actually performed using Agilent Bioanalyzer (High Sensitivity DNA kit) on the reaction mixture containing ligation molecules produced using the model DNA double strands and model Y-type adapters, it was found that the mobility varied depending on the number and position of the ligated adapters, as shown in FIG. 5. That is, it was verified that separation can be made based on the difference in the type of ligation molecules produced by the ligation reaction (complete adapter ligation molecules and incomplete adapter ligation molecules), and in other words, it was verified that the efficiency of the ligation reaction of the Y-type adapters with the DNA molecule to be analyzed can be measured with high precision by electrophoresis.

### Examples

### (Example 1)

A DNA double-strand/adapter ligation reaction was performed modeled after the actual NGS library preparation step, and the efficiency thereof was measured.

The cfDNA of interest is primarily double strands of approximately 170 bp, and the ends thereof may be 5' overhang, 3' overhang, or blunt end since they result from a reaction by deoxyribonuclease in vivo. On the other hand, the end of Y-type adapter molecules for NGS library preparation is generally equipped with a protruding T at the 3' end. Therefore, a kit for NGS library preparation generally includes two modules: one module for repairing the ends of DNA double strands and adding dA to the 3' end, and another module for the adapter ligation. Since the efficiency of the ligation of DNA double strands to adapter molecules in each kit and under each reaction condition is the results of three types of reactions by both modules, as the model DNA double strands for the model ligation test of this Example, 170 bp DNA with a 4-nt 5' overhang at one end and a 4-nt 3' overhang at the other end was used. The model DNA double strands were created by a PCR reaction with a primer having a recognition sequence for a 4-nt 5' overhang restriction enzyme and a primer having a recognition sequence for a 4-nt 3' overhang restriction enzyme, followed by a treatment of the PCR product with restriction enzymes.

As for the Y-type adapters, the Y-type adapters included in commercial kits (manufactured by Company A and Company B) that are actually used for NGS library preparation were used. Their structure is a "partially double-stranded Y-type" and basically each kit includes only one type of Y-type adapter, with a 3' overhang of a single dT nucleotide at the end of the double-stranded portion.

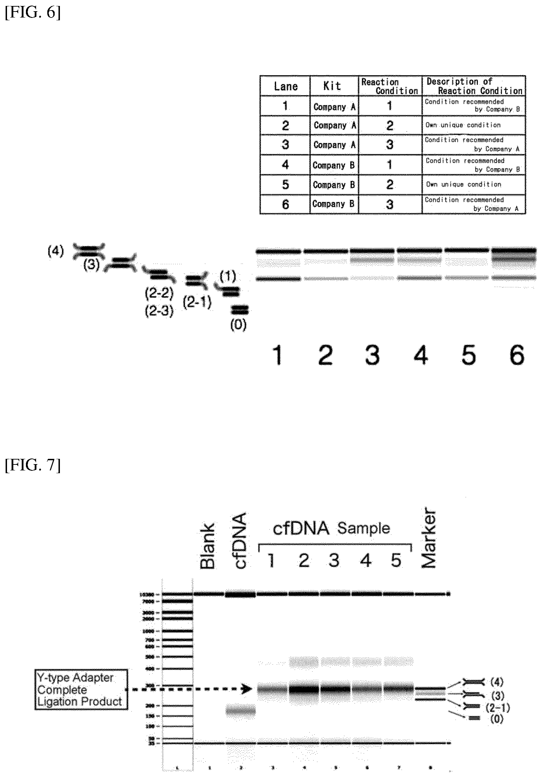

Ligation reactions using the two types of Y-type adapters were performed under the condition recommended for each kit, the condition recommended for other company's kit, and the condition modified by the inventors on their own. The resulting ligation molecules were electrophoresed and the results are shown in FIG. 6.

For each of the conditions shown in FIG. 6, the percentage of the complete adapter ligation molecules in the produced ligation molecules was as follows.

**[Table 1]**

| Lane | 1 | 2 | 3 | 4 | 5 | 6 |
|---|---|---|---|---|---|---|
| Percentage of complete ligation molecules (%) | 40 | 56 | 49 | 44 | 52 | 38 |

### (Example 2)

Using cfDNA included in a liquid biopsy, a ligation reaction of Y-type adapters was performed under the following conditions.
- cfDNA used: A DNA sample eluted in purified water using QIAamp Circulating Nucleic Acid kit by Qiagen Inc. from plasma separated within 48 hours after collecting blood in blood collection tubes by Streck Corporate.
- Reaction conditions (Reaction kit used was NEBNext Ultra II DNA Library Prep Kit for Illumina by New England Biolabs Inc.)
   1. cfDNA: 50 ng was repaired in 30 microliters, and dT was added.
   2. The above reaction product was mixed with 75 picomoles of Y-type adapters (Integrated DNA Technologies Inc.) and the ligation reaction was performed in a total of 52.5 microliters. The reaction temperature was 7°C and the reaction time was 12 hours.

The results of the electrophoresis of the cfDNA-adaptor ligation products (referred to as "cfDNA Samples" in FIG. 7) produced by the ligation reaction are shown in FIG. 7. It was confirmed that the complete ligation of the adapters was achieved with high efficiency (70% or more) under the conditions describe above.

## Claims

1. A method for evaluating efficiency of a ligation reaction that ligates Y-type adapters to both ends of DNA to be analyzed, in sequencing the DNA to be analyzed using the Y-type adapters,
wherein the efficiency of the reaction is evaluated by electrophoresing a reaction mixture containing ligation molecules comprising the DNA and the Y-type adapters produced by the ligation reaction under a specified condition, and analyzing a band separated based on the number of adapters ligated to the DNA.

2. A method for optimizing a condition for a ligation reaction, comprising the steps of:
(1) performing the ligation reaction under a first specified condition and performing the evaluation method on the reaction mixture to evaluate a first reaction efficiency; then
(2) performing the ligation reaction under a second specified condition which is at least partially modified from the first specified condition, and performing the evaluation method on the reaction mixture to evaluate a second reaction efficiency; and
(3) comparing the first reaction efficiency with the second reaction efficiency.

3. The method according to claim 2, comprising performing the steps (2) and (3) repeatedly multiple times.

## Amended claims

### Amended claims under Art. 19.1 PCT

1. [Amended] A method for evaluating a ligation reaction between a double-stranded DNA and Y-type adapters used for preparing a sample for a next-generation sequencing of DNA using the Y-type adapters, comprising the steps of:
(1) creating a model molecule that can be produced by a ligation reaction between a double-stranded DNA and Y-type adapter double DNA strands, the model molecule corresponding to each of (a) a complete adapter ligation molecule having four adapter DNA strands ligated to both ends of a double-stranded DNA, (b) an incomplete adapter ligation molecule having no adapter ligated to a double-stranded DNA, (c) an incomplete adapter ligation molecule having one adapter DNA strand ligated to a double-stranded DNA molecule, (d) an incomplete adapter ligation molecule having two adapter DNA strands ligated to a double-stranded DNA molecule, and (e) an incomplete adapter ligation molecule having three adapter DNA strands ligated to a double-stranded DNA molecule, and then performing an electrophoresis using the each model molecule to verify a method and condition enabling separation by each model molecule type based on electrophoretic mobility that varies according to the number and position of the adapter DNA strands ligated to the double-stranded DNA; and
(2) evaluating efficiency of the reaction between the double-stranded DNA to be analyzed and Y-type adapters by electrophoresing the reaction mixture from a reaction between the double-stranded DNA to be analyzed and the Y-type adapter molecules under a specified condition together with the each model molecule by applying the method and condition verified to enable the separation and identification in the step (1), determining, for each of the multiple electrophoretic bands resulting from the ligation product of the double-stranded DNA to be analyzed and Y-type adapters, the number and position of Y-type adapter molecules ligated to the double-stranded DNA to be analyzed in the reaction product corresponding to each band based on their different degrees of mobility, and also quantifying the reaction product, and calculating, based on the quantification, the amount of the reaction product of the complete adapter ligation molecule type having four Y-type adapter DNA strands ligated to the double-stranded DNA to be analyzed, and the amount of the reaction product of the incomplete adapter ligation molecule type having three or less Y-type adapter DNA strands ligated thereto.

2. [New] The method according to claim 1, wherein the model molecule is produced by reacting model DNA double strands having different 5' overhang sequences (α and β) added at both ends using Type IIS restriction enzyme with 2 types of model Y-type adapter molecules each having a 5' overhang sequence (α' or β') compatible with either of the overhang sequences (α or β) of the model DNA double strands.

3. [Amended] A method for optimizing a condition for a ligation reaction between a double-stranded DNA to be analyzed and Y-type adapters, comprising the steps of:
(A) performing a ligation reaction between the double-stranded DNA to be analyzed and the Y-type adapters under a first specified condition, and performing the step (2) of the method according to claim 1 on the reaction mixture to evaluate a first reaction efficiency; then
(B) performing a ligation reaction under a second specified condition which is at least partially modified from the first specified condition, and performing the step (2) of the method according to claim 1 on the reaction mixture to evaluate a second reaction efficiency; and
(C) comparing the first reaction efficiency with the second reaction efficiency.

4. [Amended] The method according to claim 3, comprising performing the steps (A) and (B) repeatedly multiple times.

Statement under Art. 19.1 PCT
Claim 1 of the Claims has been amended to specify in more detail the invention (method) recited in claim 1 as originally filed. Specifically, the amendment has been made to separately recite the steps of: (1) verifying by using a model molecule, based on the descriptions in paragraphs [0018] to [0027], etc. of the specification; and (2) evaluating the ligation reaction between the double-stranded DNA to be analyzed and Y-type adapter molecules, based on the descriptions in Examples, etc. of the specification.

Claim 2 of the Claims has been newly added as a claim to specify the details of the model molecule to be used in step (1) of claim 1, based on the descriptions in paragraphs [0021] and [0022] of the specification.

Claims 3 and 4 of the Claims, respectively, correspond to claims 2 and 3 as originally filed, which have been moved due to the addition of new claim 2, and have been amended to explicitly refer to step (2) of claim 1.

Meanwhile, the verifying step using a model molecule is neither disclosed nor suggested in JP2020-516281A, which has been cited in the International Search Report.
